# EUROPEAN PATENT SPECIFICATION

(11) **EP 1 373 368 B1**
(45) Date of publication and mention of the grant of the patent: **27.02.2013**
(21) Application number: 02713114.3
(22) Date of filing: 25.03.2002
(51) Int. Cl.: C08G 83/00, C08L 101/00, A61K 47/48, B82Y 5/00, C08F 8/30

(54) **COMPOUNDS FOR A CONTROLLED RELEASE OF ACTIVE COMPOUNDS**
VERBINDUNGEN ZUR GESTEUERTEN FREISETZUNG VON WIRKSTOFFEN
COMPOSES SERVANT A EFFECTUER LA LIBERATION CONTROLEE DE COMPOSES ACTIFS

(30) Priority: 27.03.2001 WO PCT/IB01/00524
(43) Date of publication of application: 02.01.2004
(73) Proprietor: Firmenich SA, 1211 Genève 8 (CH)
(72) Inventor: HERRMANN, Andreas, CH-1205 Geneva (CH); FREROT, Eric, F-74100 Ville La Grand (FR)
(74) Representative: Salvaterra-Garcia, Maria de Lurdes
(86) International application number: PCT/IB2002/000921
(87) International publication number: WO 2002/077074

(56) References cited:
- WO-A-91/07989
- US-A- 4 206 259
- US-A- 5 338 532
- US-A- 5 530 092
- JAGUR-GRODZINSKI J: "BIOMEDICAL APPLICATION OF FUNCTIONAL POLYMERS" REACTIVE & FUNCTIONAL POLYMERS, ELSEVIER SCIENCE PUBLISHERS BV, NL, vol. 39, 1999, pages 99-138, XP000826971 ISSN: 1381-5148

## Description

### Technical field

The present invention relates to the field of perfumery. More particularly, it concerns a compound having supported carbamoyl/ester moieties which comprise an ester capable of liberating an active compound and, in proximity, a carbamoyl function facilitating the release of the said active compound. The present application concerns also the uses of said compound, as well as the compositions comprising said compounds.

### Prior art

The industry has a particular interest in compounds which are capable of prolonging the effect of active compounds over a certain period of time, for example in order to overcome the problems encountered when using perfuming ingredients which are too volatile. US patent 5,649,979 in particular discloses compounds which, under certain activation conditions such as the presence of enzymes, in particular lipases, are capable of liberating a fragrance ingredient over an extended period of time. These compounds can have various applications. US 5,338,532 also relates to the release of perfuming molecules. However the compounds described in this document are different from the ones of the present invention. In particular, the groups having a structure similar to group Q of the compound of the present invention are only used during the reaction as protective groups and are not present in the final product. Contrary to what is the case in the present invention, these groups are not used to release alcohols or enols.

The washing of textiles is a particular field in which there is a constant quest to enable the effect of perfuming substances to be effective for a certain period after washing and drying. Many substances having fragrances which are particularly suitable for this type of application are, in fact, known to lack tenacity on laundry, or do not remain on the laundry when rinsed, with the result that their perfuming effect is experienced only briefly and not very intensely. Given the importance of this type of application in the perfuming industry, research in this field has been sustained, in particular with the aim of finding new, even more effective solutions to the aforementioned problems.

### Description of the invention

Surprisingly, we have discovered the existence of new compounds based on oligomer or polymer supported carbamoyl/ester moieties which comprise an ester capable of liberating active perfuming compound and, in proximity, a carbamoyl function facilitating the liberation of the said perfuming compound without the aid of an external activator, unlike what is described in the prior art. As "active compound" we mean here any perfumery alcohol or ketone or aldehyde, these latter being possibly in the enol form.

The compounds of the present invention have the following formula 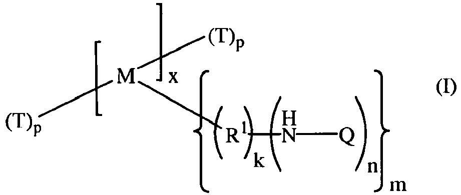 wherein
a) n represents an integer from more than zero to 4;
b) x represents an integer from 1 to 50000;
c) p represents 1 or 0 if x ≥ 2, or 1 if x = 1;
d) m represents 1 or 2 if x > 1, an integer from 2 to 4 if x =1, or an integer from 2 to 80 if M represents a dentritic core;
e) the (T)ₚ-[M-((R¹)ₖ-(NH)ₙ)ₘ]ₓ-(T)ₚ moiety represents a group derived from one of the compounds selected in the group consisting of:
   - a polyamidoamine dendrimer, a polyalkylamine dendrimer, more preferably a polypropyleneimine dendrimer such as those available under the tradename PAMAM Starburst^{®} from Dendritech Inc. or ASTRAMOL^{®} from DSM;
   - a chitosan, a polyamino alginate or cellulose, a cyclodextrine or a starch derivative containing at least two NH₂ groups, such as those commercially available from the company Carbomer;
   - a polyalkyleneimine such as the polyethyleneimine commercially available under the tradename Lupasol^{®} from BASF, Sternamines^{®} from Clariant or Jeffamine^{®} from Mitsibushi; and
   - a polylysine such as poly-DL-Lysine;
f) Q represents a hydrogen atom or a radical of the formulae 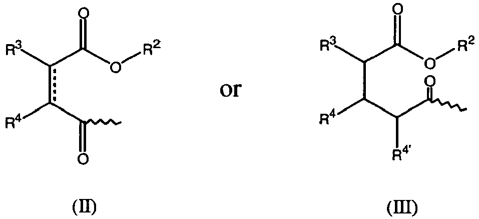
in which the wavy line indicates the location of the bond between said moiety Q and the NH group , the dotted line indicates the location of a single or double bond;
R² represents a radical derived from active perfuming alcohol or enol of the formula R²OH;
R³, R⁴ and R^{4'} represent a hydrogen atom or a C₁ to C₂₀ linear or branched, saturated or unsaturated, radical, possibly substituted and possibly comprising one or more heteroatoms; or said R³, R⁴ and R^{4'}, when considered together with the carbon atoms to which they are bonded, can form aromatic or aliphatic monocyclic, bicyclic or tricyclic groups; and with the proviso that at least two of the Q in formula (I) are not a hydrogen atom.

It is understood that whenever in a compound of formula (I) there are more than one Q group, then each said group may be identical or different to the other Q groups. The same applies to the other symbols n, m, x, p.

Groups which are possible substituents of R³, R⁴ or R^{4'} are for example hydroxyl groups, alkoxy or polyglycol groups, aromatic or alkylaromatic groups, amines and in particular quaternary ammonium functions, amides, dialkyl amides, SO₃H or OSO₃H groups, N-oxydes or carboxylic groups. Preferably, the substituents will be selected from the group comprising polyethylene or propylene glycol, polysaccharides, sulphonates and quaternary ammonium functions, e.g. of formulae xiv) to xvi): 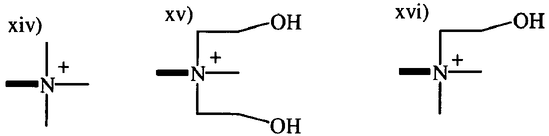 the solid line indicating the bond to said R³, R⁴or R^{4'} group.

Furthermore, the compounds of the invention wherein Q, n, x, p and m are as defined previously and the (T)ₚ[M]ₓ-(T)ₚ moiety represents a polymer derived from cellulose or an amino acid such as a natural fiber based on cellulose or an amino acid, e.g. cotton, linen, silk, viscose or paper are more preferred.

The compounds of formula (I) can be synthesized from commercially available compounds by conventional methods. Generally speaking, the compounds of the invention are susceptible of being obtainable by a process comprising the following steps:
a) reacting a diacid or an anhydride, such as a phthalic, succinic, maleic or glutaric anhydride or acid, with an active compound of formula R²OH to form a derivative containing an ester bond and a carboxylic acid function;
b) converting the carboxylic acid function obtained in step a) into an acyl chloride or fluoride or into a mixed anhydride; and
c) reacting the derivative obtained in step b) with the primary amino function of a compound of formula T-[M-(R¹)ₖ-(NH₂)ₙ)ₘ]ₓ-T, as defined hereinabove; or alternatively
d) reacting the derivative obtained in step b) with the primary amino function of a monomeric precursor of the polymeric back-bone M, as defined hereinabove; and
e) polymerize, according to any standard method, the compound obtained in step d).

An example of this approach is illustrated in the following scheme:

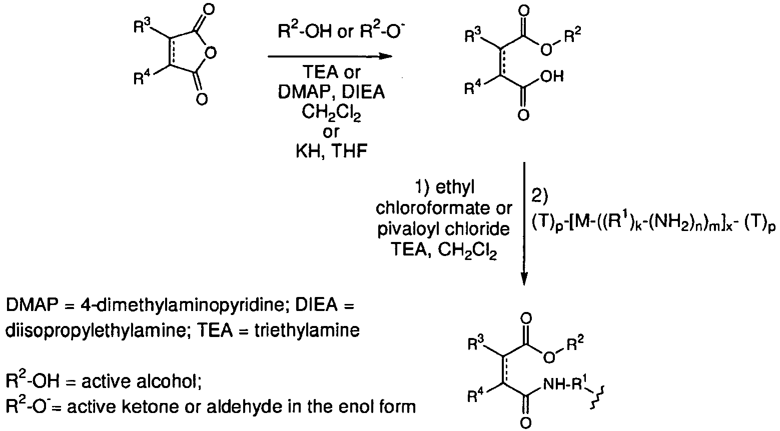

The compounds of the invention are composed of two main parts, the carrier moiety (T)ₚ-[M-((R¹)ₖ)ₘ]ₓ-(T)ₚ and the release moiety NHQ.

The carrier moiety (T)ₚ-[M-((R¹)ₖ)ₘ]ₓ-(T)ₚ plays essentially the role of support to which are attached several releasing units NHQ; however, in the case where the compounds of the invention are intended for an application implying their deposition on a surface, said carrier moiety can also play an important role in the effective deposition and surface substantivity of the compounds of formula (I), especially on fabrics. Said role in the effective deposition depends on the specific chemical nature of carrier moiety and is well known by a person skilled in the art.

The nature of R¹ plays also an important role in the fine-tuning of the release kinetics of the active compound R²OH, e.g. an alcohol. Generally, primary alcohols are more rapidly released than secondary or tertiary alcohols; however, with a suitable choice of R¹, the speed of the active alcohol release can be influenced considerably. Indeed the release of R²OH will be carried out more or less rapidly as a function of the chain length, the number of heteroatoms on the main chain, or the degree of branching of said R¹ group.

The special feature of the invention resides in the structure of the release moiety NHQ. Thanks to said particular structure, the hydrolysis of the ester group, which causes liberation of the active compound, is assisted by the nucleophilic group adjacent to the ester function, the CONH-R¹ group. This assistance has a completely unexpected advantage, i.e. it permits the cleavage of the ester bond by hydrolysis under neutral or alkaline conditions, as shown by the following scheme:

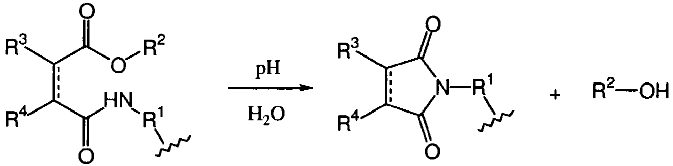

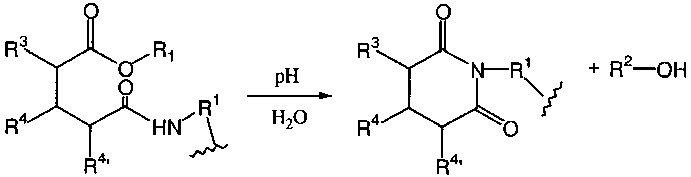

Such pH changes are, for example, the normal conditions for a washing cycle of textiles, wherein the pH may change from a value corresponding to an acid medium to values corresponding to a neutral or even basic medium during the course of the washing cycle, thus allowing the compounds according to the invention to undergo the hydrolysis process.

Furthermore, the reaction may be catalyzed naturally in the presence of heat. This is the case for example when laundry is dried, in particular in an electric dryer, or ironed, especially steam ironed. The hydrolysis reaction leads to the liberation of an active compound R²OH, in which R² has the meaning indicated hereinabove, and a residue of the initial precursor, an imide. Said residue being generally, and preferably, inactive, i.e. odorless in the case R²OH is a perfuming compound.

The reaction does not require any other external agent such as the presence of a lipase as described in the prior art.

As mentioned above, the compounds of the invention are capable of releasing odoriferous compounds of formula R²OH, i.e. perfumery alcohols or enols resulting from the ketones or aldehydes commonly used in perfumery. Although it is not possible to provide an exhaustive list of the currently known odoriferous compounds of the formula R²OH usable according to the invention, the following can be named as examples:
a) as alcohols: eugenol, 2-cyclohexyl-1-propanol, 1-decanol, geraniol, nerol, 3,7-dimethyl-1-octanol, citronellol, 1-dodecanol, ethyl vanilline, 2-ethyl-1-hexanol, 1-hexanol, pipol, vegetol, 4-hydroxy-3-methoxybenzaldehyde (vanillin), 4-(4-hydroxy-1-phenyl)-2-butanone (raspberry ketone), 7-p-menthan-1-ol (Mayol^{®}; origin: Firmenich SA, Geneva, Switzerland), anisic alcohol, guaiacol, 2-methoxy-2-phenyl-1-ethanol, isoeugenol, cyclomethylene citronellol, 2-methyl-4-phenyl-1-pentanol, 2-methyl-5-phenyl-1-pentanol, 3-methyl-5-phenyl-1-pentanol, 6-nonen-1-ol, 2,6-nonadien-1-ol, 1-octanol, 2-phenoxy-1-ethanol, 1-phenyl-1-ethanol, 2-phenyl-1-ethanol, 2-phenyl-1-propanol, 3-phenyl-1-propanol, cinnamic alcohol, salicylates, 2,4,6-trimethyl-3-cyclohexene-1-methanol, farnesol, 3,5,5-trimethyl-1-hexanol, 1-undecanol, 10-undecen-1-ol, patchone, 2-tert-butyl-4-methyl-1-cyclohexanol (rootanol), 6,8-dimethyl-2-nonanol, 4,8-dimethyl-7-nonen-2-ol, (E)-3,3-dimethyl-5-(2',2',3'-trimethyl-3'-cyclopenten-1'-yl)-4-penten-2-ol (Polysantol^{®}; origin: Firmenich SA, Geneva, Switzerland), ethyl 3-hydroxy hexanoate, 5-ethyl-2-nonanol, dartanol, 3-hydroxy-2-butanone, 1-(4-isopropyl-1-cyclohexyl)-1-ethanol, menthol, 8-p-menthen-2-ol, isopulegol, 7-methoxy-3,7-dimethyl-2-octanol, 2-methoxy-4-propyl-1-cyclohexanol (Tarragol^{®}; origin: Firmenich SA, Geneva, Switzerland), 4-methyl-3-decen-5-ol (origin: Givaudan SA, Geneva, Switzerland), 1-(4-methylphenyl)-1-ethanol (methyl paratolyl carbinol), 4-methyl-1-phenyl-2-pentanol, 1,2,3,4,4a,5,8,8a-octahydro-2,2,6,8-tetramethyl-1-naphthalenol, 3-methyl-5-(2,2,3-trimethyl-3-cyclopenten-1-yl)-2-pentanol (origin: Givaudan SA, Geneva, Switzerland), 2-octanol, 3-octanol, 1-octen-3-ol, 3,4,5,6,6-pentamethyl-2-heptanol (kohinol), 2-pentyl-1-cyclopentanol (cyclopentol), 4-phenyl-2-butanol, 4-phenyl-3-buten-2-ol, 1-phenyl-2-hexanol, 1-phenyl-2-pentanol, 1-phenyl-2-propanol, limbanol, fenchol, borneol, 3-(5,5,6-trimethyl-bicyclo[2.2.1]hept-2-yl)-1-cyclohexanol (Sandela^{®}; Givaudan), vebanol, 4-(2,6,6-trimethyl-1-cyclohexen-1-yl)-3-butenol (beta-ionol), alpha-ionol, norlimbanol, 2-undecanol, 3-benzyl-3-pentanol, 4-cyclohexyl-2-methyl-2-butanol (origin: Firmenich SA, Geneva, Switzerland), 2,6-dimethyl-2-heptanol, ethyl linalool, 3,7-dimethyl-1,6-octadien-3-ol (linalool), 3,7-dimethyl-3-octanol (tetrahydrolinalool), 2,6-dimethyl-2-octanol (tetrahydromyrcenol), 2,6-dimethyl-7-octen-2-ol (dihydromyrcenol), hydroxycitronellal, 8-p-menthanol, terpinenol, alpha-terpineol, methyl-4-phenyl-2-butanol, 2-methyl-1-phenyl-2-propanol, 2-(4-methylphenyl)-2-propanol, perhydro-4,8a-dimethyl-4a-naphthalenol (geosmin), tetrahydro-2-isobutyl-4-methyl-4(2H)-pyranol (Florol^{®}; origin: Firmenich SA, Geneva, Switzerland), linalyl oxide, 2,6,10,10-tetramethyl-1-oxaspiro[4.5]decan-6-ol (spiranol), 2,6,6,8-tetramethyl-tricyclo[5.3.1.0(1,5)]undecan-8-ol (cedrenol), nerolidol and pinanol;
b) as aldehydes susceptible to provide the enol: citral, citronellal, campholenic aldehyde, cinnamic aldehyde, hexylcinnamic aldehyde, formyl pinane, hydroxycitronellal, cuminic aldehyde, vanilline, ethyl vanilline, Lilial^{®} [3-(4-tert-butylphenyl)-2-methylpropanal ; origin : Givaudan-Roure SA, Vernier, Switzerland], Lyral^{®} [4- and 3-(4-hydroxy-4-methylpentyl)-3-cyclohexene-1-carbaldehyde ; origin : International Flavors and Fragrances, USA], Bourgeonal^{®} [3-(4-tert-butylphenyl)propanal ; origin : Quest International, Naarden, Netherlands], heliopropanal [3-(1,3-benzodioxol-5-yl)-2-methylpropanal ; origin: Firmenich SA, Geneva, Switzerland], Zestover (2,4-dimethyl-3-cyclohexene-1-carbaldehyde; origin : Firmenich SA, Geneva, Switzerland), Trifernal ^{®} (3-phenylbutanal ; origin: Firmenich SA, Geneva, Switzerland), α-sinensal, (4-methylphenoxy)acetaldehyde, 1,3-benzodioxol-5-carboxaldehyde (heliotropine), Scentenal ^{®} [8(9)-methoxy-tricyclo[5.2.1.0.(2,6)]decane-3(4)-carbaldehyde; origin: Firmenich SA, Geneva, Switzerland], Liminal ^{®} [(4R)-1-p-menthene-9-carbaldehyde ; origin: Firmenich SA, Geneva, Switzerland], Cyclosal [3-(4-isopropylphenyl)-2-methylpropanal ; origin: Firmenich SA, Geneva, Switzerland], ortho- and para-anisaldehyde, 3-methyl-5-phenylpentanal, Acropal ^{®} [4-(4-methyl-3-pentenyl)-3-cyclohexene-1-carbaldehyde ; origin : Givaudan-Roure SA., Vernier, Switzerland], Intreleven ^{®} aldehyde (mixture of 10-undecenal and 9-undecenal ; origin: International Flavors & Fragrances, USA), muguet aldehyde [(3,7-dimethyl-6-octenyl)acetaldehyde; origin: International Flavors & Fragrances, USA], 2,6-dimethyl-5-heptanal, Precyclemone ^{®} B [1-methyl-4-(4-methyl-3-pentenyl)-3-cyclohexen-1-carbaldehyde ; origin : International Flavors & Fragrances, USA] and Isocyclocitral ^{®} (2,4,6-trimethyl-3-cyclohexene-1-carbaldehyde ; origin : International Flavors & Fragrances, USA);
c) as ketones susceptible to provide the enol: camphor, carvone, menthone, ionones, irones, damascenones and damacones, benzyl acetone (4-phenyl-2-butanone), 1-carvone, 4-(4-hydroxy-1-phenyl)-2-butanone (raspberry ketone), Hedione ^{®} (methyl dihydrojasmonate; origin: Firmenich SA, Geneva, Switzerland), Neobutenone [1-(5,5-dimethyl-1-cyclohexen-1-yl)-4-penten-1-one ; origin : Firmenich SA, Geneva, Switzerland], Calone ^{®} (7-methyl-2H,4H-1,5-benzodioxepin-3-one ; origin: C.A.L. SA, Grasse, France), Sulfox [(1R,4R)-8-mercapto-3-p-menthanone ; origin: Firmenich SA, Geneva, Switzerland], Orivone^{®} [4-(1,1-dimethylpropyl)-1-cyclohexanone; origin: International Flavors & Fragrances, USA], Delphone (2-pentyl-1-cyclopentanone; origin: Firmenich SA, Geneva, Switzerland), 2-naphthalenyl-1-ethanone, Veloutone (2,2,5-trimethyl-5-pentyl-1-cyclopentanone ; origin: Firmenich SA, Geneva, Switzerland), 4-isopropyl-2-cyclohexen-1-one, Iso E Super ^{®} [isomer mixture of 1-(octahydro-2,3,8,8-tetrame-2-naphthalenyl)-1-ethanone ; origin: International Flavors & Fragrances, USA], Plicatone [5-methyl-exo-tricyclo[6.2.1.0(2,7)]undecan-4-one ; origin: Firmenich SA, Geneva, Switzerland] ; and macrocyclic ketones such as, for example Exaltone ^{®} (cyclopentadecanone), Delta-Muscenone (mixture of 3-methyl-4-cyclopentadecen-1-one and 3-methyl-5-cyclopentadecen-1-one) and muscone (3-methyl-1-cyclopentadecanone), all from Firmenich SA, Geneva, Switzerland.

The compounds according to the invention have proved to be advantageous precursors of active compounds, and in particular of fragrance ingredients. Their main advantage, as previously mentioned, is that on the one hand, they do not need an external agent and, on the other hand, the hydrolysis reaction of the ester bond, as mentioned hereinabove, can also be controlled from the kinetic point of view through the choice of the group R¹. These behaviors enable the system according to the invention to be adapted to the requirements of several possible applications and therefore represents an indisputable advantage.

Furthermore, these compounds have, generally, excellent staying-power or tenacity on a surface, especially on laundry, making them very suitable precursors in particular for applications associated with functional perfumery. Perfuming ingredients present as such in products such as washing powders or detergents can have little staying-power and be consequently often eliminated in the rinsing water during machine washing for example. Conversely, the compounds according to the invention, owing to their substantivity and the controlled liberation of the odoriferous compound, can impart a fragrance and a freshness to laundry which will last well beyond the rinsing and drying processes.

Therefore, the compound of formula (I) might be advantageously associated with compositions intended for perfuming applications. Consequently, the perfuming compositions or perfumed articles comprising a compound of formula (I) are also an object of the present invention.

Thus, the compounds according to the invention can be employed for any application requiring the effect of rapid or prolonged liberation of an odoriferous component as defined hereinabove. They can be used in particular in functional perfumery, particularly applications such as liquid or solid detergents for the treatment of textiles and/or in fabric softeners. One of the chief advantages of the invention resides in the fact that the compounds impart an intense fragrance to the laundry, produced by an odoriferous compound, which would not be detected on the laundry over a sufficiently long period if the alcohol had been used as it is, i.e. without a precursor.

The compounds according to the invention can be used as perfuming ingredients for laundry in all types of compositions, e.g. detergent or softening bases. If necessary, in some of the more aggressive media, for example basic media such as detergents, the compound of formula (I) may have to be protected from premature decomposition, for example by encapsulation.

Preferred perfuming compositions or perfumed articles are the fabric softeners, having a pH less than 7, in which these compounds are more stable.

Typical examples of fabric detergents or softener compositions into which the compounds of the invention can be incorporated are described in WO 97/34986, for the former, or in US patents 4,137,180 and 5,236,615 or EP 799 885, for the latter. Other typical detergent and softening compositions which can be used are described in works such as Ullman's Encyclopedia of Industrial Chemistry, vol. A8, pages 315 - 448 (1987) and vol. A25, pages 747 - 817 (1994); Flick, Advanced Cleaning Product Formulations, Noye Publication, Park Ridge, New Jersey (1989); Showell, in Surfactant Science Series, vol. 71: Powdered Detergents, Marcel Dekker, New York (1988); Proceedings of the World Conference on Detergents (4th, 1998, Montreux, Switzerland), AOCS print.

Naturally, the use of the compounds according to the invention is not limited to the products mentioned hereinabove. These compounds lend themselves equally well to all the other uses common in perfumery, namely the perfuming of soaps and shower or bath gels, after-shaves, hygiene products or hair care products such as shampoos or conditioner, as well as deodorants and air fresheners and also cosmetic preparations.

The compounds can also be used in applications such as detergent compositions or cleaning products for washing up or for cleaning various surfaces, whether they are intended for domestic or industrial use.

In these applications, they can be used alone, mixed together or mixed with other perfuming ingredients, solvents or additives commonly used in perfumery. The nature and type of these co-ingredients do not warrant a more detailed description here, which in any case would not be exhaustive, the skilled person being able to select them on the basis of his general knowledge and according to the nature of the product to be perfumed and the desired olfactory effect. These perfuming ingredients belong to chemical classes as varied as alcohols, aldehydes, ketones, esters, ethers, acetates, nitriles, terpene hydrocarbons, nitrogenous or sulphurous heterocyclic compounds and essential oils of natural or synthetic origin. Many of these ingredients are in any case listed in reference texts such as the book by S. Arctander, Perfume and Flavor Chemicals, 1969, Montclair, New Jersey, USA, or more recent versions thereof, or in other similar books, or yet in the specialized patent literature commonly available in the art.

The proportions in which the compounds according to the invention can be incorporated into the various aforementioned products vary within a wide range of values. These values are dependent on the nature of the article or product to be perfumed and on the desired olfactory effect as well as the nature of the co-ingredients in a given composition when the compounds according to the invention are mixed with perfuming co-ingredients, solvents or additives commonly used in the art.

For example, typical concentrations are in the order of 0.1 % to 10 % by weight, or even more, of these compounds based on the weight of the composition into which they are incorporated. Concentrations lower than these can be used when these compounds are applied directly in the perfuming of the various consumer products mentioned hereinabove.

Although special mention has been made hereinabove of the perfuming effect that can be exerted by the invention compounds, the same principles apply to analogous compounds of the invention aimed for the release of flavoring or sanitizing compounds, the perfuming compound being then replaced by a flavoring or sanitizing compound. By the term "sanitizing compound", we refer here to those substances which can exert an attractant or repellent effect towards certain species of insects, for instance towards houseflies or mosquitoes, or else, or which can have a fungicide, a bactericide or a bacteriostatic activity. It is therefore possible to have flavoring, insect repellent or attractant, bactericide or fungicide precursors of the formula (I), or yet compositions, products or articles containing them. In these applications, they can be used alone, mixed together or mixed with other active ingredients, solvents or additives commonly used in the respective art.

It goes without saying that mixtures of such agents can also be used.

The invention also relates to a process for intensifying or prolonging the diffusion effect of an active compound in a surface, characterized in that said surface is contacted with a compound of formula (I). Preferably said surface is a textile and the compound of formula (I) is contained in a detergent and/or a fabric softener.

Yet another object of the invention is the use of a compound of formula (I) as an active ingredient, e.g. perfuming ingredient, or as a precursor capable of liberating an active alcohol, ketone or aldehyde or a mixture thereof.

Apart from the above-mentioned applications and methods of use, some of the compounds of formula (I) may also be used as active fibers, which are useful for the manufacture of, for example, active papers or cloths. As "active fiber" we mean here a fiber capable of bringing a benefit or effect into its surrounding environment, and in particular a perfumery, insect repellent or attractant, bactericide or fungicide form.

Indeed, this is the case when the (T)ₚ-[M]ₓ-(T)ₚ moiety represents a polymer or a fiber derived from cellulose or an amino acid, e.g. cotton, linen, silk, viscose or paper. In such a case, the active compound precursor is the fiber itself in which the (R¹)ₖ(NHQ)ₙ moiety is chemically bonded. The fiber is therefore able to impart the desired benefit without any additional treatment such as a washing cycle, as it is the case in the above-mentioned application in detergents.

The active compound R²OH can be thus liberated upon use of the fabrics, and for example counteract unpleasant odors such as perspiration. Such fabrics or papers may also be useful as, e.g., ambient air-fresheners or insect repellent or attractant pads.

The invention will now be described in further detail by way of the following examples, wherein the abbreviations have the usual meaning in the art, the temperatures are indicated in degrees centigrade (°C) ; the NMR spectral data were recorded in CDCl₃ at 360MHz for ¹H and at 90.6 MHz for ¹³C (if not stated otherwise), the chemical displacement δ is indicated in ppm with respect to TMS as standard, the coupling constants J are expressed in Hz and all the abbreviations have the usual meaning in the art. UV/V is spectra were recorded on a Perkin-Elmer Lambda 14 instrument, IR spectra on a Perkin Elmer 1600 FTIR spectrometer.

Commercially available reagents and solvents were used without further purification if not stated otherwise. Reactions were carried out in standard glassware under N₂. Column chromatography: Silica gel 60 A (35-70 microns) from SDS. HPLC analyses were carried out on a Thermo Separation Products apparatus consisting of an online vacuum degasser, a SpectraSystem P4000 quaternary pump, a SpectraSystem AS3000 autosampler thermostatted at 20°C and a SpectraSystem UV6000LP diode array detector coupled with a SEDERE Sedex 55 evaporative light scattering detector. The amount of free NH₂ groups (in mmol/g of resin) of the unmodified and modified polymers described below was determined using the Gisin test described in the literature (B. F. Gisin, Anal. Chim. Acta 1972, 58, 248-249). Dendrimers and their derivatives are named according to G. R. Baker and J. K. Young in Advances in Dendritic Macromolecules, Vol. 1, 1994, G. R. Newcome (Ed.), JAI Press Inc., Greenwich, Connecticut, USA.

### Example 1 (not according to the invention)

### Preparation of (6E,11Z,19Z,24E)-2,6,25,29-tetramethyl-9,22-dioxa-14,17-diaza-2,6,11,19,24,28-triaconta-hexaene-10,13,18, 21-tetrone

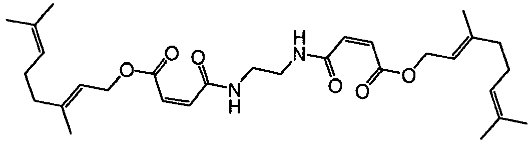

Geraniol (3.08 g, 20 mmol) and maleic anhydride (1.98 g, 20 mmol) were dissolved in 40 ml of CH₂Cl₂. Triethylamine (3 ml, 20 mmol) was added and the reaction was stirred overnight at room temperature. The monogeranyl maleate which was formed was used as such in the next step. Triethylamine (3 ml, 20 mmol) was added to the reaction mixture and the temperature was lowered to -20°C. Pivaloyl chloride (2.66 g, 22 mmol) was added dropwise and the reaction was stirred for 2 h at room temperature. 1,2-Ethylene diamine (0.70 ml, 10 mmol) in 20 ml of CH₂Cl₂ was added dropwise during 45 minutes. The reaction was stirred for another 3.5 h. Then, the resulting solution has been acidified using an aqueous solution of KHSO₄ (5%). The organic phase was washed with water 3 times, dried over Na₂SO₄ and concentrated. Column chromatography (SiO₂, cyclohexane/ethyl acetate 3:7) gave 1.43 g (yield = 14%) of an oil.
IR (neat, cm⁻¹): 3281 (m), 3091 (w), 2962 (w), 2913 (m), 2874 (w), 2849 (w), 1724 (s), 1669 (m), 1654 (m), 1630 (m), 1566 (s), 1455 (m), 1438 (m), 1401 (m), 1375 (m), 1315 (w), 1266 (s), 1243 (w), 1225 (s), 1164 (s), 1108 (m), 1067 (w), 1010 (m), 990 (m), 957 (m), 882 (m), 836 (m), 821 (w), 802 (m), 768 (m), 730 (m), 691 (m), 653 (m).
¹H-NMR: 7.65 (*s*, 2 H); 6.44 (*d, J* = 12.5, 2 H); 6.06 (*d, J* = 12.5, 2 H); 5.32 (*t, J* = 7.1, 2 H); 5.07 (*m,* 2 H); 4.62 (*d, J* = 7.1, 4 H); 3.56 (*d,* br., 4 H); 2.14-2.02 (*m*, 8 H); 1.70 (s, 6 H); 1.68 (s, 6 H); 1.60 (s, 6 H).
¹³C-NMR: 165.6 (*s*); 143.4 (*s*); 138.8 (*d*); 132.0 (*s*); 124.1 (d); 123.6 (*d*); 117.3 (*d*); 62.2 (*t*); 39.6 (*t*); 39.0 (*t*); 29.3 (*t*); 25.7 (*q*); 17.7 (*q*); 16.5 (*q*).
MS (ESI): 551 ([M+Na]⁺, 100).

### Example 2

### a) Preparation of 1-benzylbutyl hydrogen phthalate

4.85 g (32.7 mmol) of phthalic anhydride were added in portions into 70 ml of CH₂Cl₂ containing 5.38 g (32.7 mmol) of 1-phenyl-2-pentanol, 0.40 g (3.3 mmol) of 4-dimethylaminopyridine (DMAP) and 4.23 g (3.3 mmol) of diisopropylethylamine (DIEA). The reaction mixture was left stirring at room temperature for 24 h, then poured onto 100 ml of aqueous KHSO₄ (5%), and extracted with 50 ml of aqueous KHSO₄ (5%) and 3 times with 50 ml of brine. The organic phase was dried over Na₂SO₄ and concentrated to give 9.94 g (yield = 97%) of a slightly yellow oil, which slowly crystallizes.
IR (neat, cm⁻¹): 3060 (w), 3030 (w), 2960 (m), 2931 (m), 2903 (m), 2871 (m), 2662 (m), 2541 (m), 1726 (s), 1685 (s), 1599 (m), 1578 (m), 1492 (m), 1454 (m), 1436 (m), 1412 (m), 1350 (m), 1282 (s), 1256 (s), 1200 (m), 1170 (w), 1146 (m), 1119 (s), 1073 (s), 1059 (m), 1038 (m,) 1014 (m), 985 (m), 950 (s), 872 (w), 848 (w), 836 (w), 824 (m), 801 (m), 777 (m), 748 (m), 731 (s), 690 (s), 682 (s).
¹H-NMR: 10.64 (br., 1 H); 7.91-7.84 (*m,* 1 H); 7.58-7.43 (*m*, 3 H); 7.33-7.17 (*m,* 5 H); 5.40-5.30 *(m,* 1H), 3.06 (*ABX, J =* 13.9, 6.7, 1 H); 2.90 (*ABX, J =* 13.5, 6.7, 1 H); 1.74-1.25 (*m*, 4 H); 0.89 (*t, J =* 7.3, 3 H).
¹³C-NMR: 172.4 (*s*); 167.7 (*s*); 137.5 (*s*); 133.9 *(s);* 132.1 (*d*); 130.6 (*d*); 129.8 (*d*); 129.7 (*s*); 129.5 (*d*); 129.4 (*d*); 128.6 (*d*); 128.4 (*d*); 126.5 (*d*); 76.6 (*d*); 40.2 (*t*); 35.3 (*t*); 18.6 (*t*), 13.9 (*q*).
MS (EI): 313 ([M+H]⁺, 0.4), 167 (13), 150 (9), 149 (100), 147 (11), 146 (84), 121 (10), 117 (31), 115 (3), 105 (5), 104 (18), 103 (3), 93 (11), 92 (49), 91 (28), 77 (4), 76 (3), 65 (13), 55 (7), 41 (3), 39 (4).

### b) Preparation of (f)-bis(1-benzylbutyl) 2,2'-(6-methyl-1,11-dioxo-2,6,10-triazaundecane-1, 11-diyl)dibenzoate

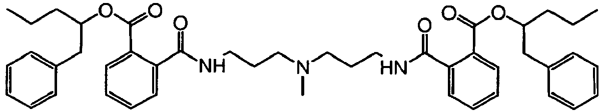

A solution of 1.00 g (3.2 mmol) of 1-benzylbutyl hydrogen phthalate and 0.65 g (6.4 mmol) of triethylamine in 10 ml of CH₂Cl₂ was cooled to 0°C, before 0.35 g (3.2 mmol) of ethyl chloroformate in 5 ml of CH₂Cl₂ were added dropwise during 5 minutes. The reaction mixture was left stirring at 0°C for 10 minutes and then 0.21 g (1.4 mmol) of 3,3'-methylamino-bis(propanamine) in 5 ml of CH₂Cl₂ were added. Finally, another 0.05 g (0.5 mmol) of ethyl chloroformate in 2 ml of CH₂Cl₂ were added after 10 min followed by 0.12 g (1.3 mmol) of *N,N*-dimethylethylenediamine in 3 ml of CH₂Cl₂ 15 min later. The mixture was kept stirring for 15 min at 0°C, then poured into 50 ml of aqueous KHSO₄ (5%) and ice and extracted with 50 ml of CH₂Cl₂. The organic phase was washed 10 times with 50 ml of aqueous KHSO₄ (5%), dried over Na₂SO₄, filtered, concentrated and dried under high vacuum to give 1.11 g (yield = 99%) of a white solid.
UV/Vis (water/acetonitrile 2:1): 305 (sh, 200), 282 (sh, 1600), 275 (1900), 268 (1900), 264 (1900).
IR (neat, cm⁻¹): 3275 (m), 3060 (w), 3026 (w), 2955 (w), 2928 (w), 2866 (w), 1771 (w), 1712 (m), 1650 (m), 1595 (m), 1579 (m), 1538 (m), 1494 (w), 1482 (m), 1463 (m), 1453 (m), 1396 (w), 1362 (w), 1287 (m), 1261(m), 1161 (w), 1126 (m), 1078 (m), 1045 (m), 1028 (m), 962 (w), 944 (w), 849 (w), 782 (w), 737 (m), 699 (m).
¹H-NMR: 7.86 (m, 2 H); 7.57-7.45 (m, 4 H); 7.40-7.12 (m, 14 H); 5.26-5.15 (m, 2 H); 3.48-3.28 (m, 4 H); 3.27-3.12 (m, 1 H); 3.05-2.83 (m, 3 H); 3.01 (ABX, J = 13.5, 5.9, 2 H); 2.87 (ABX, J = 13.5, 6.3, 2 H); 2.55 (s, 3 H); 2.08-1.90 (m, 4 H); 1.68-1.20 (m, 8 H); 0.87 (t, J = 7.3, 6 H).
¹³C-NMR: 169.4 (s); 167.0 (s); 137.6 (s); 137.4 (s); 131.3 (s); 131.1 (d); 129.6 (d); 129.5 (d); 129.0 (d); 128.542 (d); 128.4 (d); 128.0 (d); 126.4 (d); 75.8 (d); 53.9 (t); 40.2 (t); 39.7 (q); 36.5 (t); 35.4 (t); 23.7 (t); 18.6 (t); 14.0 (q).
MS (EI): 121 (4), 103 (6), 93 (9), 92 (100), 91 (46), 77 (6), 73 (6), 71 (3), 70 (6), 65 (11), 61 (5), 57 (4), 55 (17), 51 (3), 45 (8), 44 (3), 43 (42), 41 (7), 40 (3), 39 (6).

### Example 3

### a) Preparation of (1,1-dimethyl-2-phenylethyl) hydrogen phthalate

8.0 g of potassium hydride (20% in oil, 39.9 mmol) were washed with pentane and THF. Then 10 ml of fresh THF were added and 5 g of 2-methyl-1-phenyl-2-propanol (33.3 mmol) in 10 ml of THF were added dropwise during 15 minutes. The resulting solution was stirred for 1 h at room temperature. Consequently, the reaction mixture was added dropwise over 15 minutes to a mechanically stirred solution of 4.93 g (33.3 mmol) of phthalic anhydride, 4.30 g (33.3 mmol) of DIEA and 0.40 g (3.3 mmol) of DMAP in 200 ml of THF. At the end of the introduction another 50 ml of THF were added and the reaction mixture was heated to 50°C and left cooling to room temperature during 1 hour. The reaction mixture was poured into a stirred mixture of 300 ml of ice and 200 ml of aqueous KHSO₄ (5%). 200 ml of ether were added and the organic phase extracted twice with 100 ml of aqueous KHSO₄ (5%) and 3 times with brine. Extraction with 100 ml of a saturated aqueous solution of NaHCO₃ (2 times), treatment of the aqueous phase with 25 g of KHSO₄, re-extraction twice which 100 ml of ether, washing twice with 50 ml of aqueous KHSO₄ (5%), drying and concentration gave 7.42 g (yield = 75%) of white crystals.
IR (neat, cm⁻¹): 2973 (w), 2919 (w), 2866 (w), 2661 (w), 2556 (w), 1716 (m), 1689 (s), 1596 (m), 1578 (m), 1490 (m), 1467 (w), 1452 (m), 1415 (m), 1383 (m), 1370 (m), 1310 (m), 1285 (s), 1268 (s), 1237 (m), 1207 (w), 1186 (w), 1140 (m), 1114 (s), 1068 (s), 1031 (w), 1009 (w), 968 (w), 938 (m), 910 (w), 866 (w), 844 (m), 833 (m), 806 (w), 792 (m), 772 (m), 737 (s), 724 (m), 699 (s), 684 (m), 670 (w).
¹H-NMR: 11.55 (br., 1 H); 7.91-7.85 (m, 1 H); 7.62-7.47 (m, 3 H); 7.29-7.15 (m, 5 H); 3.17 (s, 2 H); 1.58 (s, 6 H).
¹³C-NMR: 172.9 (s); 167.3 (s); 137.0 (s); 135.0 (s); 132.1 (d); 130.7 (d); 130.3 (d); 129.7 (d); 129.6 (s); 128.6 (d); 128.0 (d); 126.5 (d); 84.5 (s); 46.7 (t); 25.5 (q).
MS (EI): 167 (3), 150 (9), 149 (100), 147 (3), 146 (21), 133 (5), 132 (37), 122 (3), 121 (9), 118 (3), 117 (25), 115 (9), 105 (8), 104 (15), 93 (10), 92 (12), 91 (27), 77 (6), 76 (8), 73 (3), 70 (4), 65 (18), 61 (5), 59 (10), 57 (3), 55 (4), 51 (4), 50 (5), 45 (5), 44 (3), 43 (19), 41 (4), 39 (6).

### b) Preparation of bis(1,1-dimethyl-2-phenylethyl) 2,2'-(6-methyl-1,11,dioxo-2,6,10-triazaundecane-1,11-diyl)dibenzoate

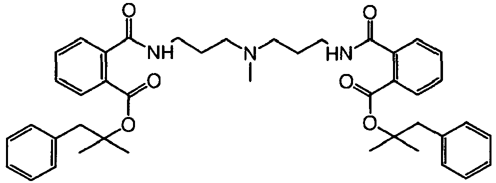

This compound was synthesized as described in Example 2b with 1.00 g (3.4 mmol) of (1,1-dimethyl-2-phenylethyl) hydrogen phthalate, 0.68 g (6.7 mmol) of triethylamine in 20 ml of CH₂Cl₂, 0.36 g (3.4 mmol) of ethyl chloroformate in 5 ml of CH₂Cl₂, 0.21 g (1.4 mmol) of 3,3'-methylamino-bis(propanamine) in 5 ml of CH₂Cl₂ and another 0.06 g (0.5 mmol) of ethyl chloroformate in 1 ml of CH₂Cl₂ followed by 0.12 g (1.3 mmol) of *N,N*-dimethylethylenediamine in 3 ml of CH₂Cl₂ to give 1.03 g (yield = 97%) of a slightly yellow solid.
UV/Vis (water/acetonitrile 2:1): 274 (2200), 268 (sh, 2300), 264 (2400).
IR (neat, cm⁻¹): 3270 (m, br.), 3059 (w), 3027 (w), 2976 (w), 2924 (w), 2873 (w), 1708 (m), 1641 (m), 1596 (m), 1578 (m), 1537 (m), 1494 (w), 1481 (m), 1469 (m), 1452 (m), 1442 (m), 1383 (m), 1368 (m), 1290 (m), 1256 (m), 1215 (m), 1176 (m), 1182 (m), 1112 (m), 1084 (m), 1043 (m), 1030 (m), 970 (m), 889 (w), 847 (m), 829 (w), 788 (w), 773 (w), 730 (m), 701 (m).
¹H-NMR: 7.89 (t, J = 5.7, 2 H); 7.52-7.44 (m, 4 H); 7.35-7.13 (m, 14 H); 3.45-3.29 (m, 4 H); 3.29-3.13 (m, 2 H); 3.10 (s, 4 H); 3.02-2.87 (m, 2 H); 2.52 (s, 3 H); 2.07-1.92 (m, 4 H); 1.48 (s, 12 H).
¹³C-NMR: 169.5 (s); 166.9 (s); 137.3 (s); 137.0 (s); 132.3 (s); 131.0 (d); 130.7 (d); 129.4 (d); 129.0 (d); 128.0 (d); 127.9 (d); 126.5 (d); 83.6 (s); 53.9 (t); 46.5 (t); 39.6 (q); 36.5 (t); 25.8 (q); 23.8 (t).
MS (EI): 150 (3), 148 (3), 135 (16), 132 (7), 117 (14), 115 (7), 104 (4), 93 (8), 92 (100), 91 (52), 89 (4), 77 (4), 76 (3), 65 (14), 63 (4), 59 (48), 58 (3), 57 (8), 51 (5), 50 (3), 44 (3), 43 (18), 41 (7), 39 (8).

### Example 4 (not according to the invention)

### Preparation of bis(1,1-dimethyl-2-phenylethyl) 2,2'-(1,11-dioxo-2,10-diazaundecane-1,11-diyl)dibenzoate

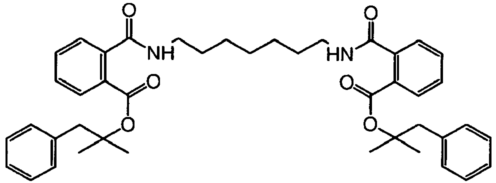

This compound was synthesized as described in Example 2b with 2.00 g (6.7 mmol) of (1,1-dimethyl-2-phenylethyl) hydrogen phthalate (prepared as described in Example 3a), 1.35 g (13.4 mmol) of triethylamine in 20 ml of CH₂Cl₂, 0.73 g (6.7 mmol) of ethyl chloroformate in 10 ml of CH₂Cl₂, 0.39 g (3.0 mmol) of 1,7-diaminoheptane in 10 ml of CH₂Cl₂ (added at room temperature) and another 0.11 g (1.0 mmol) of ethyl chloroformate in 5 ml of CH₂Cl₂ followed by 0.11 g (1.2 mmol) of *N,N*-dimethylethylenediamine in 5 ml of CH₂Cl₂. The mixture was kept stirring for 15 min, then poured into 50 ml of aqueous KHSO₄ (5%), stirred for 1 h and extracted with 30 ml of CH₂Cl₂. The organic phase was washed six times with 20 ml of aqueous KHSO₄ (5%), dried over Na₂SO₄, filtered, concentrated and dried under high vacuum. Plug filtration of the crude reaction product on 50 g of reversed phase RP C4 silica gel (*Vydac*^{®}) with water/acetonitrile 1:1 then pure acetonitrile (both containing 0.1% of TFA) gave, after drying under high vacuum, 1.36 g (yield = 29%) of a highly viscous yellow oil.
UV/Vis (water/acetonitrile 2:1): 273 (6800), 264 (sh, 7300), 258 (sh, 9000).
IR (neat, cm⁻¹): 3281 (m, br.), 3063 (w), 3027 (w), 2930 (m), 2854 (w), 2251 (w), 1777 (w), 1711 (s), 1640 (s), 1596 (w), 1576 (w), 1537 (s), 1493 (w), 1482 (w), 1467 (w), 1452 (m), 1443 (m), 1383 (m), 1368 (m), 1289 (s), 1256 (m), 1208 (s), 1158 (s), 1113 (s), 1081 (m), 1039 (w), 1030 (w), 974 (w), 917 (w), 890 (w), 846 (m), 801 (w), 772 (m), 729 (s), 701 (s).
¹H-NMR: 7.77-7.71 (m, 2 H); 7.52-7.35 (m, 6 H); 7.30-7.15 (m, 10 H); 6.06 (t, J = 5.7, 2 H); 3.34 (q, J = 6.6, 4 H); 3.19 (s, 4 H); 1.97 (s, 4 H); 1.54 (s, 12 H); 1.36 (s, 6 H).
¹³C-NMR: 170.1 (s); 166.0 (s); 137.5 (s); 137.1 (s); 131.5 (d); 130.8 (s); 130.6 (d); 129.96 (d); 129.6 (d); 128.0 (d); 127.8 (d); 126.5 (d); 84.1 (s); 46.1 (t); 40.1 (t); 29.2 (t); 28.8 (t); 26.7 (t); 25.9 (q).
MS (ESI): 693 (11), 692 (41), 691 ([M+H]⁺, 100), 560 (6), 559 (16), 428 (5), 427 (18).

### Example 5

### a) Preparation of (±)-(1,5-dimethyl-1-vinyl-4-hexenyl) hydrogen phthalate

8.74 g of potassium hydride (20% in oil, 43.6 mmol) were washed with pentane and THF. Then 20 ml of THF were added and 6.17 g of linalol (40.0 mmol) in 10 ml of THF were added dropwise during 15 min. The reaction mixture was left stirring for 30 min at room temperature and then added dropwise during 15 minutes to a mechanically stirred solution of 5.40 g (36.4 mmol) of phthalic anhydride, 4.70 g (36.4 mmol) of DIEA and 0.44 g (3.6 mmol) of DMAP in 250 ml of THF and the reaction mixture was left stirring at room temperature for 18 hours. The reaction mixture was poured on 300 ml of ice and 400 ml of aqueous KHSO₄ (5%) were added and extracted with 300 ml of ether. The aqueous phase was re-extracted with 100 ml of ether. The combined organic phases were washed with 100 ml of aqueous KHSO₄ (5%), concentrated and taken up in 50 ml of ether. The solution was then treated with 20 ml of a solution of aqueous NaHCO₃ (5%) and washed twice with 20 ml of brine. Drying over Na₂SO₄ and concentrating gave 10.63 g (yield = 91%) of a slightly orange oil.
IR (neat, cm⁻¹): 3070 (m), 2971 (m), 2910 (m), 2855 (m), 2649 (m), 2534 (m), 1692 (s), 1597 (m), 1580 (m), 1490 (m), 1450 (m), 1405 (m), 1375 (m), 1283 (s), 1259 (s), 1173 (w), 1126 (m), 1071 (s), 1038 (w), 1003 (w), 993 (w), 973 (w), 919 (m), 855 (w), 796 (m), 737 (m), 702 (w), 695 (w), 672 (m).
¹H-NMR: 8.87 (br., 1 H); 7.90-7.83 (m, 1 H); 7.74-7.68 (m, 1 H); 7.63-7.49 (m, 2 H); 6.13 (dd, J = 17.4, 11.1, 1 H); 5.24 (d, J = 17.4, 1 H); 5.19 (d, J = 11.5, 1 H); 5.13-5.05 (m, 1 H); 2.10-1.79 (m, 4 H); 1.70 (s, 3 H); 1.63 (s, 3 H); 1.56 (s, 3 H).
¹³C-NMR: 172.5 (s); 166.5 (s); 141.2 (d); 134.1 (s); 131.9 (s,d); 130.6 (d); 130.3 (s); 129.7 (d); 129.0 (d); 123.7 (d); 113.7 (t); 85.2 (s); 40.1 (t); 25.6 (q); 23.0 (q); 22.41 (t); 17.6 (q).
MS (EI): 167 (9), 152 (9), 150 (9), 149 (91), 146 88), 137 (8), 136 (35), 123 (4), 122 (9), 121 (51), 111 (6), 109 (6), 108 (4), 107 (9), 105 (10), 96 (3), 95 (7), 94 (17), 93 (100), 92 (18), 91 (8), 83 (3), 82 (3), 81 (11), 80 (47), 79 (11), 77 (5), 71 (4), 69 (22), 68 (12), 67 (14), 65 (16), 59 (4), 55 (15), 53 (17), 51 (4), 45 (4), 43 (8), 41 (38), 39 (14).

### b) Preparation of (±)-bis(1,5-dimethyl-1-vinyl-4-hexenyl) 2,2'-(6-methyl-1,11-dioxo-2,6,10-triazaundecane-1,11-diyl)dihenzoate

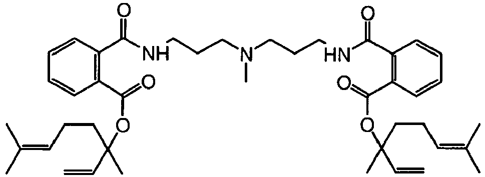

This compound was synthesized as described in Example 2b with 0.20 g (0.66 mmol) of (±)-(1,5-dimethyl-1-vinyl-4-hexenyl) hydrogen phthalate and 0.14 g (1.39 mmol) of triethylamine in 10 ml of CH₂Cl₂, 0.075 g (0.69 mmol) of ethyl chloroformate in 5 ml of CH₂Cl₂, 0.090 g (0.62 mmol) of 3,3'-methylamino-bis(propanamine) in 5 ml of CH₂Cl₂ and another 0.011 g (0.1 mmol) of ethyl chloroformate in 5 ml of CH₂Cl₂ followed by 0.050 g (0.55 mmol) of N,N-dimethylethylenediamine in 3 ml of CH₂Cl₂ 20 min later. The mixture was kept stirring for 10 min at 0°C, then poured into 20 ml of aqueous KHSO₄ (5%) and left stirring at room temperature for 1 hour. The organic phase was washed 10 times with 50 ml of aqueous KHSO₄ (5%), dried over Na₂SO₄, filtered, concentrated and dried under high vacuum to give 0.14 g (yield = 32%) of the compound.
UV/Vis (water/acetonitrile 2:1): 283 (sh, 1900), 274 (2400).
¹H-NMR: 7.97-7.86 (t, J = 5.5, 2 H); 7.70-7.61 (m, 2 H); 7.54-7.45 (m, 2 H); 7.43-7.31 (m, 4 H); 6.05 (dd, J = 17.4, 11.1, 2 H); 5.14 (d, J = 17.8, 2 H); 5.15-5.00 (m, 2 H); 5.09 (d, J = 11.1, 2 H); 3.50-3.33 (m, 4 H); 3.31-3.08 (m, 2 H); 3.07-2.84 (m, 2 H); 2.57 (s, 3 H); 2.14-1.95 (m, 8 H); 1.94-1.73 (m, 4 H); 1.66 (s, 6 H); 1.62 (s, 6 H); 1.56 (s, 6 H).
¹³C-NMR: 169.5 (s); 166.1 (s); 141.8 (d); 137.6 (s); 131.9 (s, 2x), 131.1 (d); 129.4 (d); 129.1 (d); 128.0 (d); 123.8 (d); 113.2 (t); 84.3 (s); 53.8 (t); 40.5 (t); 39.5 (q); 36.42 (t); 25.68 (q); 23.71 (t); 23.27 (q); 22.38 (t); 17.65 (q).
MS (ESI): 715 ([M+H]⁺, 12), 714 (M⁺, 12), 713 (90), 712 (100), 709 (3), 708 (3), 706 (5), 705 (3), 704 (4), 702 (3), 579 (4), 578 (20), 576 (3), 559 (3), 443 (3), 442 (3), 441 (20), 439 (3), 434 (3), 433 (3), 410 (5), 408 (7), 406 (54), 405 (27), 404 (3), 396 (3), 376 (8), 375 (18), 243 (4), 242 (6), 241 (11), 240 (40).

### Example 6

### a) Preparation of 1,1-dimethyl-2-phenylethyl 2-(fluorocarbonyl)benzoate

A solution of 3.00 g (10.1 mmol) of (1,1-dimethyl-2-phenylethyl) hydrogen phthalate (prepared as described in Example 3*a*) and 0.80 g (10.1 mmol) of pyridine in 25 ml of CH₂Cl₂ was cooled down to -20°C before 1.60 g (12.1 mmol) of cyanuric fluoride in 5 ml of CH₂Cl₂ were added dropwise during 10 min. The reaction mixture was left stirring at -20°C for 30 min and 1 h at room temperature, filtered and rinsed with 20 ml of dichloromethane. The filtrate was washed with 60 ml of water. The aqueous phase was extracted twice with 20 ml of CH₂Cl₂. The organic phases were dried over Na₂SO₄ and concentrated. Column chromatography (SiO₂, heptane/ether 4:1) and drying (0.2 mbar, 30 min) gave 2.37 g (yield = 78%) of a slightly yellow oil.
IR (neat, cm⁻¹): 3061 (w), 3028 (w), 2978 (w), 2928 (w), 1816 (s), 1712 (s), 1598 (m), 1578 (m), 1493 (m), 1468 (w), 1452 (m), 1385 (m), 1369 (m), 1289 (s), 1269 (s), 1215 (s), 1232 (s), 1177 (m), 1138 (m), 1108 (s), 1090 (s), 1043 (m), 1029 (w), 1005 (s), 914 (w), 890 (w), 863 (w), 845 (m), 826 (w), 777 (m), 759 (m), 772 (s), 699 (s), 673(m).
¹H-NMR: 7.80-7.75 (m, 1 H); 7.75-7.69 (m, 1 H); 7.67-7.54 (m, 2 H); 7.31-7.17 (m, 5 H); 3.21 (s, 2 H); 1.60 (s, 6 H).
¹³C-NMR: 165.6 (s); 159.5 (s); 136.8 (s); 134.6 (s); 133.1 (d); 131.1 (d); 130.6 (d); 130.1 (d); 129.4 (d); 128.1 (d); 126.6 (s); 125.9 (s); 85.3 (s); 46.4 (t); 25.6 (q).
MS (CI): 319 (13), 318 ([M+NH₄]⁺, 68), 185 (3), 184 (10), 183 (100), 169 (3), 168 (31), 167 (6), 166 (54), 93 (5).

### b) Preparation of dendrimer 4-cascade:1,4-diaminobutane[4-N,N,N',N']:N-(2-[(1,1-dimethyl-2-phenylethoxy)carhonyl]benzoyl)propylamine

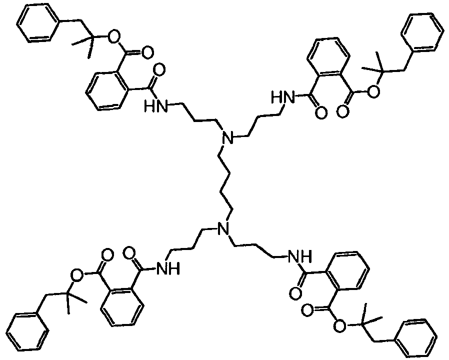

A solution of 0.077 g (0.24 mmol) of dendrimer 4-cascade:1,4-diaminobutane[4-N,N,N',N']:propylamine (Astramol^{®}-Am-4, origin: DSM) in 2 ml of dichloromethane was added dropwise during 5 min to a stirred solution of 0.35 g (1.17 mmol) of freshly prepared 1,1-dimethyl-2-phenylethyl 2-(fluorocarbonyl)benzoate and 0.235 g (2.33 mmol) of triethylamine in 6 ml of dichloromethane at 0°C. After stirring at 0°C for 2 h, the reaction mixture was left warming up to room temperature and extracted with 10 ml of aqueous KHSO₄ (5%). The aqueous phase was re-extracted twice with 5 ml of CH₂Cl₂ and the organic phases dried over Na₂SO₄. Column chromatography (RP-C4 (*Vydac*^{®} *214TP C4),* water/acetonitrile 1:1 containing 0.1% of TFA) and drying under high vacuum gave 0.163 g (yield = 47%) of the target compound.
IR (neat, cm⁻¹): 3306 (w, br.), 3061 (w), 3027 (w), 2979 (w), 2936 (w), 2872 (w), 1772 (m), 1706 (s), 1658 (m), 1596 (m), 1579 (w), 1542 (m), 1468 (w), 1452 (m), 1440 (m), 1397 (w), 1385 (m), 1368 (m), 1301 (m), 1288 (m), 1257 (w), 1201 (s), 1140 (s), 1116 (s), 1085 (s), 1042 (w), 1030 (w), 972 (w), 907 (s), 845 (m), 797 (m), 776 (m), 721 (s), 700 (s).
¹H-NMR: 7.70-7.62 (m, 4H); 7.47-7.33 (m, 8H); 7.33-7.08 (m, 28H); 3.52-3.20 (m, 16H); 3.19-2.98 (m, 4H); 3.08 (s, 8H); 2.08-1.89 (m, 8H); 1.89-1.70 (m, 4H); 1.47 (s, 24H).
¹³C-NMR: 172.4 (s); 165.9 (s); 136.8 (s); 136.4 (s); 131.9 (d); 130.6 (d); 130.5 (s); 130.0 (d, 2x); 128.1 (d); 127.5 (d); 126.7 (d); 84.2 (s); 51.4 (t); 50.7 (t); 46.5 (t); 36.7 (t); 25.3 (q); 23.8 (t); 20.5 (t).
MS (ESI): 1437.7 ([M+H]⁺).

### Example 7

### Preparation of dendrimer 8-cascade:1,4-diaminobutane[4-N,N,N',N']:1-azabutylidene: N-(2-[(1,1-dimethyl-2-phenylethoxy)carbonyl]benzoyl)propylamine

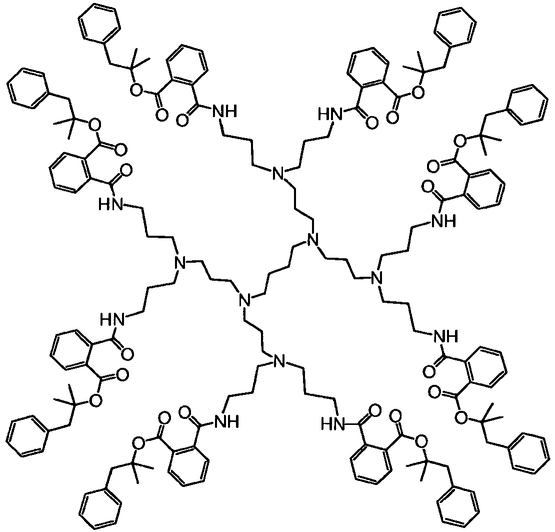

A solution of 0.36 g (0.47 mmol) of dendrimer 8-cascade:1,4-diaminobutane[4-N,N,N',N'1:1-azabutylidene:propylamine (Astramol^{®}-Am-8, origin: DSM) in 10 ml of dichloromethane was added dropwise during 10 min to a stirred solution of 1.34 g (4.46 mmol) of freshly prepared 1,1-dimethyl-2-phenylethyl 2-(fluorocarbonyl)benzoate (see Example 6a) and 0.90 g (8.92 mmol) of triethylamine in 20 ml of dichloromethane at room temperature. After stirring at room temperature for 30 min, the reaction mixture was poured into 50 ml of aqueous KHSO₄ (5%) and ice and extracted with 20 ml of CH₂Cl₂. The aqueous phase was re-extracted with 20 ml of CH₂Cl₂ and the organic phases dried over Na₂SO₄. Column chromatography (RP-C4 (*Vydac*^{®} *214TP C4),* water/acetonitrile 1:1, then pure acetonitrile, both containing 0.1% of TFA) and drying under high vacuum gave 0.40 g (yield = 28%) of the target compound.
IR (neat, cm⁻¹): 3274 (w, br.), 3062 (w), 3026 (w), 2977 (w), 2932 (w), 2871 (w), 1775 (m), 1706 (m), 1649 (m), 1596 (m), 1578(w), 1536 (m), 1470 (w), 1453 (m), 1443 (m), 1384 (m), 1369 (m), 1300 (m), 1289 (m), 1258 (w), 1198 (s), 1161 (s), 1136 (s), 1114 (s), 1085 (s), 1044(m), 1030 (w), 974 (w), 890 (w), 845 (m), 796 (m), 779 (w), 773 (w), 730 (m), 720 (m), 701 (s), 663 (w).
¹H-NMR: 7.66-7.56 (m, 8H); 7.51-7.08 (m, 72H); 3.52-3.29 (m, 32H); 3.29-3.10 (m, 20H); 3.05 (s, 16H); 2.47-2.21 (m, 8H); 2.18-1.94 (m, 16H); 1.87-1.69 (m, 4H); 1.44 (s, 48H).
¹³C-NMR: 172.6 (s); 165.8 (s); 136.76 (s); 135.9 (s); 131.8 (d); 130.68 (s); 130.6 (d); 130.1 (d); 130.0 (d); 128.1 (d); 127.6 (d); 126.65 (d); 84.2 (s); 52.2 (t); 51 (t); 50.2 (t); 49.6 (t); 46.5 (t); 37.0 (t); 25.6 (q); 23. 5 (t); 20.4 (t); 18.8 (t).
MS (ESI): 3014.6 ([M+H]⁺), 1508.4 ([M+2H]²⁺), 1005.6 ([M+3H]³⁺).

### Example 8

### Preparation of dendrimer 16-cascade:1,4-diaminobutane[4-N,N,N',N']:(1-azabutylidene)²:N-(2-[(1,1-dimethyl-2-phenylethoxy)carbonyl]benzoyl)propylamine

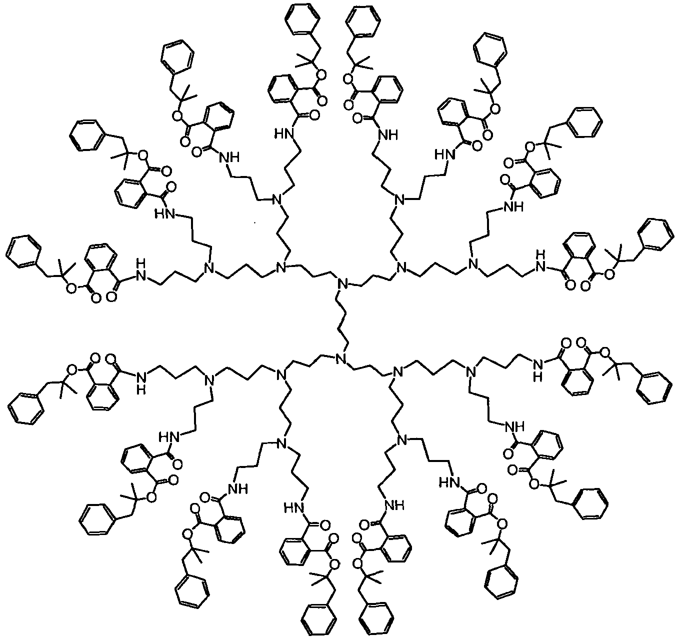

This compound was synthesized as described in Example 7 with 0.33 g (0.20 mmol) of dendrimer 16-cascade:1,4-diaminobutane[4-N,N,N',N']:(1-azabutylidene)²:propylamine (Astramol^{®}-Am-16, origin: DSM) in 10 ml of dichloromethane, 1.05 g (3.50 mmol) of freshly prepared 1,1-dimethyl-2-phenylethyl 2-(fluorocarbonyl)benzoate (see Example 6a) and 0.71 g (6.99 mmol) of triethylamine in 20 ml of dichloromethane. The reaction mixture was poured into 30 ml of aqueous KHSO₄ (5%), extracted and dried over Na₂SO₄. MPLC (RP-C4 (*Vydac*^{®} *214TP C4*), water/acetonitrile 1:1 then 1:4, containing 0.1% of TFA) with addition of 0.015 g of KHSO₄ to the product fraction gave, after concentration and lyophilisation, 0.19 g (yield *ca.* 15%) of a white solid.
IR (neat, cm⁻¹): 3294 (m, br.), 3601 (w), 2952 (m, br.), 2869 (w), 2647 (w, br.), 1777 (m), 1707 (s), 1665 (s), 1649 (s), 1596 (m), 1578 (m), 1540 (m), 1469 (m), 1453 (m), 1384 (m), 1369 (m), 1292 (s), 1259 (w), 1199 (s), 1173 (s), 1139 (s), 1118 (s), 1086 (s), 1044 (m), 980 (m), 920 (w), 889 (w), 847 (m), 798 (m), 817 (w), 778 (w), 772 (w), 761 (w), 730 (m), 720 (m), 702 (s), 676 (w).
¹H-NMR (400 MHz): 7.9-6.7 (*m*, 160 H); 4.0-2.6 (*m*, br., 116 H); 3.05 (*s*, br., 32 H); 2.6-1.0 (*m*, br., 60 H); 1.44 (*s*, br., 96 H).
¹³C-NMR (100.6 MHz): 171.4 (*s*); 166.2 (*s*); 136.9 (*s*); 136.7 (*s*); 131.6 (*d*); 131.1 (*s*); 130.6 (*d*); 129.8 (*d*); 129.7 (*d*); 128.1 (*d*); 127.6 (*d*); 126.6 (*d*); 84.1 (*s*); 50.9 (*t*, br.); 49.9 (*t*, br.); 49.0 (*t*, br.); 46.5 (*t*); 36.8 (*t*); 25.7 (*q*); 23.7 (*t*); 18.5 (*t*, br.).
MS (ESI): 2058.0 ([M+3H]³⁺), 1543.8 ([M+4H]⁴⁺), 1235.3 ([M+5H]⁵⁺).

### Example 9

### Measurement of the fragrance release by HPLC

Buffer solutions were prepared by dissolving two phosphate or borate buffer tablets (origin: Fluka, Switzerland) in a mixture of 160 ml water and 40 ml acetonitrile, respectively. To determine the exact pH value of the reaction solution, 10 ml of the buffer solutions were diluted with 2 ml of acetonitrile and the pH values measured (on a *Mettler Toledo MP220* apparatus with an *InLab 410* Ag/AgCl glass electrode) to be 7.62 (phosphate buffer) and 10.47 (borate buffer).

### a) Amount of cyclisation of the precursor under alkaline hydrolysis conditions

0.2 ml of a solution of 35 to 45 mg of the precursors in 25 ml of acetonitrile were added to 1.0 ml of a buffer solution at 20°C. The mixture was immediately injected in a HPLC apparatus (t = 0), eluted at 1 ml/min on a reversed phase column with a mixture of water/acetonitrile containing 0.1 % of TFA. Then the decrease of the amount of precursor and the simultaneous formation of the cyclized products was monitored at different time intervals at λ = 254 and 280 nm. Chromatograms were recorded on a *Macherey-Nagel, Nucleosil*^{®} *100-5 C18* column (250 x 4 mm i.d.) using a water/acetonitrile gradient (70:30 to 20:80 during 20 min) or on a *Merck Chromolith*^{™} *SpeedROD RP-C18e* column (50 x 4.6 mm i.d.) using a water/acetonitrile gradient (70:30 to 40:60 during 3 min). The results are summarized in the following table:

| Compound of Example N° | pH | Time [h] | Amount of cyclized precursor* |
|---|---|---|---|
| 1 | 7.62 | 4.7 | 97 % |
| 2b | 7.62 | 2.1 | 98 % |
| *3b* | 7.62 | 22.4 | 69 % |
| 4 | 10.47 | 3.0 | 98 % |
| *5b* | 7.62 | 9.4 | 72 % |
| 6*b* | 7.62 | 24.6 | 80 % |

| | | | |
|---|---|---|---|
| * determined for the first step of cyclisation only, % relative to the total amount of the starting compound | | | |

All compounds tested released the desired perfumery alcohol. The rate of release can be influenced by the choice of the pH, by the type of alcohol to be released as well as by the nature of the substrate linked to the carbamoyl moiety.

### b) Amount of perfumery alcohols released by alkaline hydrolysis

About 25 mg of the precursors were placed in 1 ml of a buffer solution, 0.2 ml of acetonitrile was added and the supernatant solution was immediately injected in a HPLC apparatus (t = 0), eluted at 3 ml/min on a reversed phase column with a mixture of water/acetonitrile containing 0.1 % of TFA. The amount of the released alcohol was monitored by HPLC analysis at different time intervals at λ = 214 or 254 nm. An external calibration curve for the corresponding alcohol in acetonitrile has been obtained from 4-8 dilutions. Chromatograms were recorded on a *Merck Chromolith*^{™} *Performance RP-C18e* column (100 x 4.6 mm i.d.) using a water/acetonitrile gradient (100:0 for 0.2 min then to 0:100 during 10 min or 80:20 to 20:80 during 10 min). The results are summarized in the following table:

| Compound of Example N° | pH | Time [d] | Amount of released alcohol * |
|---|---|---|---|
| 7 | 10.47 | 2 | 29% |
| 8 | 10.98**) | 4 | 69 % |
| 9c | 10.47 | 3 | 3% |
| 10b | 10.47 | 3 | 42 % |
| 11c | 10.47 | 5 | 30 % |
| 12 | 10.47 | 3 | 74% |
| 13 | 10.47 | 3 | 74% |

| | | | |
|---|---|---|---|
| * % relative to the total amount of the starting compound ** phosphate-bicarbonate buffer in water/acetonitrile 16:9. | | | |

It was verified that the polymer beads (compounds of Examples 9-13) did not contain free geraniol by washing the polymer (10 mg) with pure acetonitrile (0.5 ml). As for dimeric 2-carbamoyl precursors (see under *a*), it is possible to tune kinetic release through the nature of the substrate connected to the carbamoyl moiety from a slow release in a hydrophobic environment to a fast release in a more hydrophilic environment. The good accessibility of water molecules close to the precursor moiety is thus a very important factor for the speed of fragrance release. This could be achieved either by the presence of an hydrophilic spacer between the precursor and the polymer or by an hydrophilic arm near the precursor moiety.

### Example 10

### Measurement of fragrance release by UV spectroscopy

2 ml of a precursor solution (35-55 mg in 25 ml of acetonitrile, at 20°C) were added, at t = 0, to 10 ml of a phosphate buffer solution (prepared as described in Example 14). Hydrolysis was followed by recording the absorption spectra of the reaction mixture at constant time intervals in the range of 260-360 nm with a scanning rate of 960 nm/min until completion of the hydrolysis.

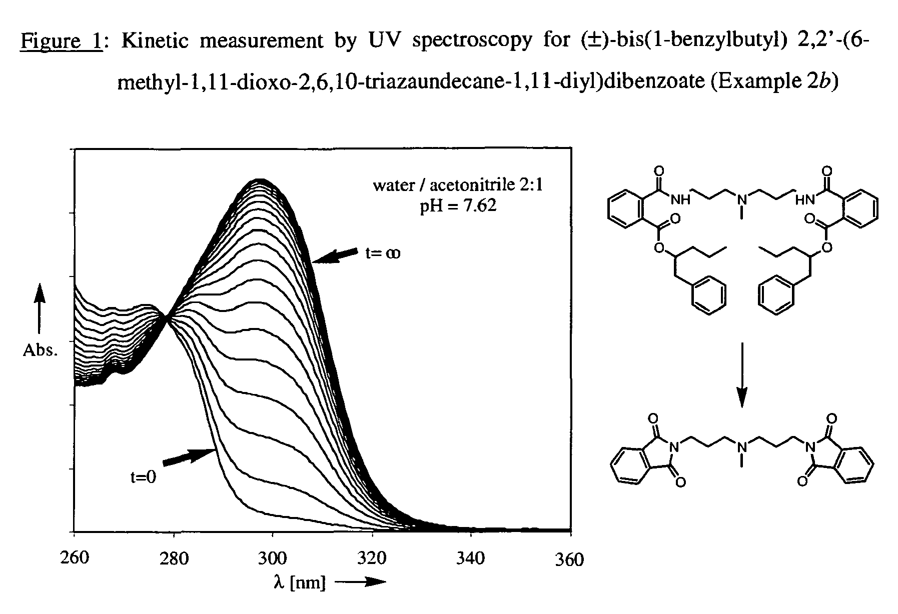

Using the same method the hydrolysis of the following compounds was also verified for bis(1,1-dimethyl-2-phenylethyl) 2,2'-(6-methyl- 1, 11-dioxo-2,6,10-triazaundecane-1, 11 - diyl)dibenzoate (Example 3b), bis(1,5-dimethyl-1-vinyl-4-hexenyl) 2,2'-(6-methyl-1,11-dioxo-2,6,10-triazaundecane-1,11-diyl)dibenzoate (Example 5b) and dendrimer 4-cascade:1,4-diaminobutane[4-N,N,N',N']:N-(2-[(1,1-dimethyl-2-phenylethoxy) carbonyl] benzoyl)propylamine (Example 6b).

### Example 11

### Release properties of grafted polymers under ambient conditions

The polymers prepared in Examples 12 and 13 are able to release geraniol upon simple exposure to ambient humidity of the air at room temperature. Thus, after 100 days they released 8% and 33% of geraniol, respectively, as measured by HPLC as described in Example 14*b*.

## Claims

1. A compound of formula 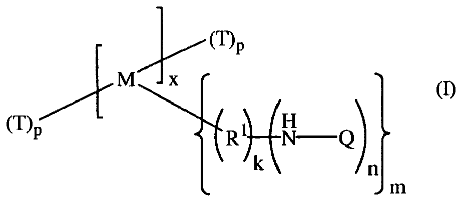 wherein
a) n represents an integer from more than zero to 4;
b) x represents an integer from 1 to 50000;
c) p represents 1 or 0 if x ≥ 2, or 1 if x = 1;
d) m represents 1 or 2 if x > 1, an integer from 2 to 4 if x =1, or an integer from 2 to 80 if M represents a dentritic core;
e) the (T)ₚ-[M-((R¹)_{d}NH)ₙ)ₘ]ₓ-(T)ₚ moiety represents a group derived from one of the compounds selected in the group consisting of:
- a polyamidoamine dendrimer, a polyalkylamine dendrimer;
- a chitosan, a polyamino alginate or cellulose, a cyclodextrine or a starch derivative containing at least two NH₂ groups;
- a polyalkyleneimine; and
- a polylysine;
f) Q represents a hydrogen atom or a radical of the formulae 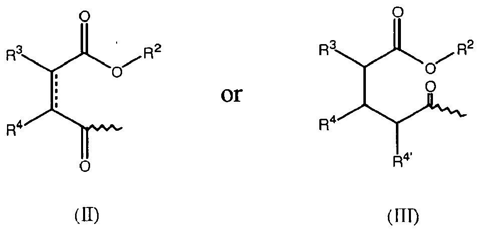 in which the wavy line indicates the location of the bond between said moiety Q and the NH group , the dotted line indicates the location of a single or double bond;
R² represents a radical derived from a perfuming alcohol or enol of the formula R²OH;
R³, R⁴ and R^{4'} represent a hydrogen atom or a C₁ to C₂₀ linear or branched, saturated or unsaturated, radical, possibly substituted and possibly comprising one or more heteroatoms; or said R³, R⁴ and R^{4'}, when considered together with the carbon atoms to which they are bonded, can form aromatic or aliphatic monocyclic, bicyclic or tricyclic groups; and with the proviso that at least two of the Q in formula (I) are not a hydrogen atom;
capable of liberating a perfuming alcohol or enol of formula R²OH, wherein R² has the same meaning as above.

2. A compound according to claim 1, **characterized in that** the (T)ₚ-[M]ₓ(T)ₚ moiety represents a natural fiber based on polyamino cellulose.

3. A compound according to claim 1, **characterized in that** the polyalkylamine dendrimer is a polypropyleneimine dendrimer, the polyalkyleneimine is a polyethyleneimine and the polylysine is a poly-DL-Lysine.

4. Use of a compound as defined in any one of claims 1 to 3 as perfuming ingredient or as a precursor capable of liberating an active alcohol, ketone or aldehyde or a mixture thereof.

5. A perfumery composition, product or articles comprising as active ingredient a compound as defined in any one of claims 1 to 3 together with a current active ingredient, solvent or adjuvant.

6. A composition, product or articles according to claim 5, **characterized in that** the residue of said compound after liberation of the active compound is inactive.

7. A composition, product or article according to claim 5 or 6, in the form of an after-shave lotion, a soap, a bath or shower gel, a shampoo or conditioner or other hair care product, a deodorant or air freshener, a cosmetic preparation, a hygiene product, a detergent or fabric softener or a cleaning product.

8. A process for intensifying or prolonging the diffusion effect of a perfuming compound in a surface, **characterized in that** said surface is contacted with a compound of formula (I) as defined in any one of claims 1 to 3.

9. A process according to claim 8, **characterized in that** said surface is a textile and the compound of formula (I) is contained in a detergent and/or a fabric softener.

## Patentansprüche

1. Verbindung der Formel 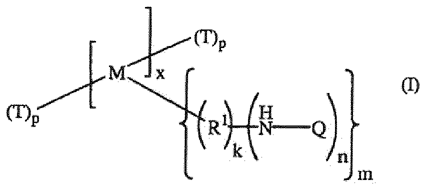 wobei
a) n eine ganze Zahl von mehr als null bis 4 darstellt;
b) x eine ganze Zahl von 1 bis 50000 darstellt;
c) p 1 oder 0, wenn x ≥ 2, oder 1, wenn x = 1, darstellt;
d) m 1 oder 2, wenn x > 1, eine ganze Zahl von 2 bis 4, wenn x = 1, oder eine ganze Zahl von 2 bis 80 darstellt, wenn M einen dendritischen Kern darstellt;
e) die (T)ₚ-[M-((R¹)ₖ-(NH)ₙ)ₘ]ₓ-(T)ₚ-Einheit eine Gruppe darstellt, abgeleitet von einer der Verbindungen, ausgewählt in der Gruppe, bestehend aus:
- einem Polyamidoamin-Dendrimer, einem Polyalkylamin-Dendrimer;
- einem Chitosan, einem Polyaminoalginat oder Cellulose, einem Cyclodextrin-oder einem Stärkederivat, enthaltend mindestens zwei NH₂-Gruppen;
- einem Polyalkylenimin; und
- einem Polylysin;
f) Q ein Wasserstoffatom oder einen Rest der Formeln 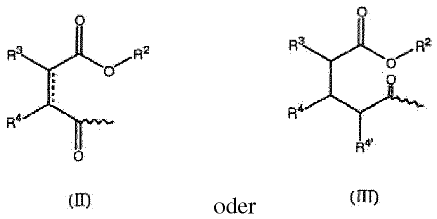 darstellt, in welchen die Wellenlinie den Ort der Bindung zwischen der Einheit Q und der NH-Gruppe anzeigt, die gestrichelte Linie den Ort einer einfachen oder doppelten Bindung anzeigt;
R² einen Rest, abgeleitet von einem parfümierenden Alkohol oder Enol der Formel R²OH, darstellt;
R³, R⁴ und R^{4'} ein Wasserstoffatom oder einen linearen oder verzweigten, gesättigten oder ungesättigten C₁- bis C₂₀-Rest, möglicherweise substituiert und möglicherweise umfassend ein oder mehrere Heteroatome, darstellen; oder das R³, R⁴ und R^{4'}, wenn zusammen mit den Kohlenstoffatomen betrachtet, an welchen sie gebunden sind, aromatische oder aliphatische monocyclische, bicyclische oder tricyclische Gruppen bilden können; und mit der Maßgabe, dass mindestens zwei von den Q in Formel (I) nicht ein Wasserstoffatom sind;
fähig zum Freisetzen eines parfümierenden Alkohols oder Enols der Formel R²OH, wobei R² die gleiche Bedeutung wie vorstehend hat.

2. Verbindung gemäß Anspruch 1, **dadurch gekennzeichnet, dass** die (T)p-[M]ₓ-(T)ₚ-Einheit eine natürliche Faser, basierend auf Polyaminocellulose, darstellt.

3. Verbindung gemäß Anspruch 1, **dadurch gekennzeichnet, dass** das Polyalkylamin-Dendrimer ein Polypropylenimin-Dendrimer ist, das Polyalkylenimin ein Polyethylenimin ist und das Polylysin ein Poly-DL-lysin ist.

4. Verwendung einer Verbindung, wie definiert in einem der Ansprüche 1 bis 3, als parfümierender Bestandteil oder als ein Vorläufer, fähig zum Freisetzen eines aktiven Alkohols, Ketons oder Aldehyds oder eines Gemisches davon.

5. Zusammensetzung, Produkt oder Gegenstände für die Parfümfabrikation, umfassend als aktiven Bestandteil eine Verbindung, wie definiert in einem der Ansprüche 1 bis 3, zusammen mit einem geläufigen aktiven Bestandteil, Lösungsmittel oder Hilfsstoff.

6. Zusammensetzung, Produkt oder Gegenstände gemäß Anspruch 5, **dadurch gekennzeichnet, dass** der Rest der Verbindung nach der Freisetzung der aktiven Verbindung inaktiv ist.

7. Zusammensetzung, Produkt oder Gegenstand gemäß Anspruch 5 oder 6 in der Form einer Aftershavelotion, einer Seife, eines Bade- oder Duschgels, eines Shampoo oder Festigers oder anderen Haarpflegeprodukts, eines Deodorant oder Luftverbesserers, einer kosmetischen Zubereitung, eines Hygieneprodukts, eines Detergens oder Weichspülers oder eines Reinigungsprodukts.

8. Verfahren zum Intensivieren oder Verlängern der Diffusionswirkung einer parfümierenden Verbindung in einer Oberfläche, **dadurch gekennzeichnet, dass** die Oberfläche in Kontakt mit einer Verbindung der Formel (I), wie definiert in einem der Ansprüche 1 bis 3, gebracht wird.

9. Verfahren gemäß Anspruch 8, **dadurch gekennzeichnet, dass** die Oberfläche eine Textilie ist und die Verbindung der Formel (I) in einem Detergens und/oder einem Weichspüler enthalten ist.

## Revendications

1. Composé de formule 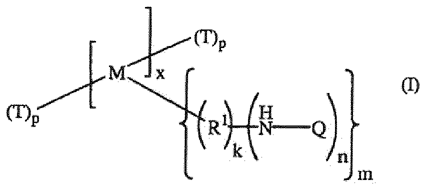 dans laquelle
a) n représente un entier de plus de zéro à 4;
b) x représente un entier de 1 à 50000;
c) p représente 1 ou 0 si x ≥ 2, ou 1 si x = 1;
d) m représente 1 ou 2 si x > 1, un entier de 2 à 4 si x = 1, ou un entier de 2 à 80 si M représente un noyau dendritique;
e) le groupement (T)ₚ-[M-((R¹)ₖ-(NH)ₙ)ₘ]_{X}-(T)ₚ représente un groupe dérivé d'un des composés choisis dans le groupe constitué de:
- un dendrimère de polyamidoamine, un dendrimère de polyalkylamine;
- un chitosane, un polyaminoalginate ou une polyaminocellulose, une cyclodextrine ou un dérivé d'amidon contenant au moins deux groupes NH₂;
- une polyalkylèneimine; et
- une polylysine;
f) Q représente un atome d'hydrogène ou un radical de formule 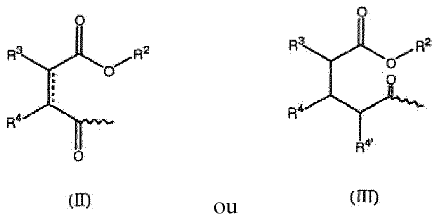 où le trait ondulé indique l'emplacement de la liaison entre ledit groupement Q et le groupe NH, le trait en pointillé indique l'emplacement d'une liaison simple ou double;
R² représente un radical dérivé d'un alcool ou énol parfumant de formule R²OH;
R³, R⁴ et R^{4'} représentent un atome d'hydrogène ou un radical en C₁ à C₂₀ linéaire ou ramifié, saturé ou insaturé, éventuellement substitué et comprenant éventuellement un ou plusieurs hétéroatomes; ou lesdits R³, R⁴ et R^{4'}, lorsqu'ils sont pris ensemble avec les atomes de carbone auxquels ils sont liés, peuvent former des groupes aromatiques ou aliphatiques monocycliques, bicycliques ou tricycliques; et à condition qu'au moins deux des Q dans la formule (I) ne soient pas un atome d'hydrogène;
capable de libérer un alcool ou énol parfumant de formule R²OH, R² ayant la même signification que ci-dessus.

2. Composé selon la revendication 1, **caractérisé en ce que** le groupement (T)ₚ-[M]ₓ-(T)ₚ représente une fibre naturelle à base de polyaminocellulose.

3. Composé selon la revendication 1, **caractérisé en ce que** le dendrimère de polyalkylamine est un dendrimère de polypropylèneimine, la polyalkylèneimine est une polyéthylèneimine et la polylysine est une poly-DL-lysine.

4. Utilisation d'un composé selon l'une quelconque des revendications 1 à 3 en tant qu'ingrédient parfumant ou en tant que précurseur capable de libérer un alcool actif, une cétone active ou un aldéhyde actif ou un de leurs mélanges.

5. Composition, produit, ou articles de parfumerie comprenant en tant qu'ingrédient actif un composé selon l'une quelconque des revendications 1 à 3 avec un ingrédient actif, solvant, ou adjuvant d'usage courant.

6. Composition, produit, ou articles selon la revendication 5, **caractérisés en ce que** le résidu dudit composé après libération du composé actif est inactif.

7. Composition, produit, ou article selon la revendication 5 ou 6, sous la forme d'une lotion après-rasage, d'un savon, d'un gel de bain ou de douche, d'un shampooing ou d'un après-shampooing ou autre produit de soin capillaire, d'un déodorant ou d'un désodorisant d'air ambiant, d'une préparation cosmétique, d'un produit d'hygiène, d'un détergent ou d'un adoucissant pour textile ou d'un produit de nettoyage.

8. Procédé pour intensifier ou prolonger l'effet de diffusion d'un composé parfumant dans une surface, **caractérisé en ce que** ladite surface est mise en contact avec un composé de formule (I) selon l'une quelconque des revendications 1 à 3.

9. Procédé selon la revendication 8, **caractérisé en ce que** ladite surface est un textile et le composé de formule (I) est contenu dans un détergent et/ou un adoucissant pour textile.
